# EUROPÄISCHE PATENTANMELDUNG

(11) **EP 0 548 751 A1**
(43) Veröffentlichungstag der Anmeldung: **30.06.1993**
(21) Anmeldenummer: 92121308.8
(22) Anmeldetag: 15.12.1992
(51) Int. Cl.: G01N 27/416

(54) **Gasspurenmesssystem**

(30) Priorität: 27.12.1991 DE 4143092
(71) Anmelder: Compur Monitors Sensor Technology GmbH, D-81539 München (DE)
(72) Erfinder: Braden, Christoph, Dr., W-5000 Köln 41 (DE)
(74) Vertreter: Wagner, Karl H., Dipl.-Ing.

(57) **Zusammenfassung**

Das Gasspurenmeßsystem besteht aus dem Gassensor 2 und dem eigentlichen Meßgerät (Auswerteelektronik) 1, das auch die Anzeige 8 enthält. Der Gassensor 2 umfaßt neben der Meßzelle 3 einen elektronischen nicht flüchtigen Speicher 4, in dem meßzellenspezifische Daten gespeichert sind. Diese Daten können von einem Rechner 7 im Meßgerät 1 ausgelesen und weiterverarbeitet werden. Damit wird die Voraussetzung geschaffen, daß der Rechner 7 beim erstmaligen Betrieb des Gasspurenmeßsystems die Empfindlichkeit und die Nullpunktverschiebung aus dem Speicher 4 abfragt und eine automatische Kalibrierung des Gerätes durchführt.

## Beschreibung

Die Erfindung geht aus von einem Gasspurenmeßsystem, das einen Gassensor und eine Auswerteelektronik mit Anzeige umfaßt. Der Gassensor besteht bei den bisher bekannten Gasspurenmeßsystemen aus einer Meßzelle, die ein mit der zu bestimmenden Gaskonzentration korreliertes elektrisches Meßsignal erzeugt. Als Meßzellen werden üblicherweise elektrochemische Zellen oder Halbleiterzellen auf der Basis einer Leitfähigkeitsmessung verwendet Üblicherweise sind die Meßzelle und die Auswerteelektronik in ein komplettes Meßgerät integriert.

Zur Erkennung und Spurenanalyse von toxischen Gasen werden sehr häufig Gassensoren auf der Basis von elektrochemischen Zellen eingesetzt. Ein Nachteil dieser Gassensoren ist die begrenzte Lebensdauer (typisch 1 bis 2 Jahre), was einen hohen Wartungs- und Serviceaufwand verursacht, Wegen der relativ starken Exemplarstreuung dieser Meßzellen ist eine optimale Auflösung nur durch individuelle Kalibrierung (Nullwert, Empfindlichkeit) der einzelnen Meßzellen erreichbar. Bei jedem Meßzellenwechsel muß das Meßgerät manuell auf die aus dem beigelegten Prüfprotokoll entnommenen Kalibrierdaten der neuen Meßzelle eingestellt werden, Bei Meßgeräten, die mit unterschiedlichen Meßzellentypen ausgerüstet werden können, ist zusätzlich der Sensortyp einzustellen. Diese sich immer wiederholenden Einstellungsarbeiten sind zeitintensiv und bringen eine hohe Verwechselungsgefahr von Meßzellentypen und Meßzellendaten mit sich, Während des Meßzellenwechsels ist zusätzlich die kontinuierliche Überwachung der Gaskonzentration unterbrochen, was unter Umständen mit Produktionsausfällen verbunden ist.

Der Erfindung liegt daher die Aufgabe zugrunde ein Gasspurenmeßsystem zu entwickeln, bei dem ohne großen Aufwand eine Überprüfung der Meßzellentypen und Meßzellendaten erfolgen kann. Die Überprüfung soll von der Auswerteelektronik nach Möglichkeit selbsttätig durchgeführt werden.

Diese Aufgabe wird bei einem aus einem Gassensor und einer Auswerteelektronik bestehenden Gasspurenmeßsystem erfindungsgemäß dadurch gelöst, daß der Gassensor neben der Meßzelle einen elektronischen nicht flüchtigen Speicher enthält, in dem meßzellenspezifische Daten gespeichert sind und daß die Auswerteschaltung einen Rechner umfaßt, der die meßzellenspezifischen Daten ausliest und weiterverarbeitet.

Zweckmäßig ist der Gassensor mit der Meßzelle und dem Speicher als Austauschmodul ausgebildet.

In dem Speicher sollen zumindest Informationen über den Meßzellentyp hinsichtlich der zu messenden Gaskomponente, die Empfindlichkeit und die Nullpunktsverschiebung abgelegt sein.

Vorteilhaft wird von dem Rechner beim erstmaligen Betrieb des Gasspurenmeßsystems die Empfindlichkeit und die Nullpunktsverschiebung aus dem Speicher abgefragt und eine automatische Kalibrierung des Gerätes durchgeführt.

Gemäß einer bevorzugten Ausführungsform besteht der Gassensor aus einem Austauschmodul mit einer elektrochemischen Meßzelle, deren Kenndaten aus dem im Austauschmodul integrierten elektronischen Speicher abrufbar sind.

Mit der Erfindung werden folgende Vorteile erzielt:
- Die vom Hersteller mitgelieferten Kalibrierdaten können in dem im Sensor integrierten Speicher abgelegt werden und sind auf diese Weise unverlierbar und eindeutig an die Meßzelle gebunden.
- Eine Fehlkalibrierung und die daraus resultierende Fehlfunktion des Gasspurenmeßsystems ist ausgeschlossen.
- Die Aktualisierung bereits vorhandener Meßgeräte durch Vorgabe neuer Schwellwerte, z.B. von Alarmschwellen, ist in einfacher Weise möglich.
- Im Speicher kann auch die alterungsbedingte Abnahme der Meßzellenempfindlichkeit (Empfindlichkeitsdrift) abgelegt sein. Unter Berücksichtigung des ebenfalls gespeicherten letzten Kalibrierdatums kann dann dieser Alterungseffekt durch den Rechner kompensiert werden.
- Bei der Ausführung mit automatischer Selbstkalibrierung beim erstmaligen Betrieb des Gasspurenmeßsystems kann der Austausch des Sensormoduls auch von ungeschultem Personal durchgeführt werden und erfordert nur einen sehr geringen Serviceaufwand.

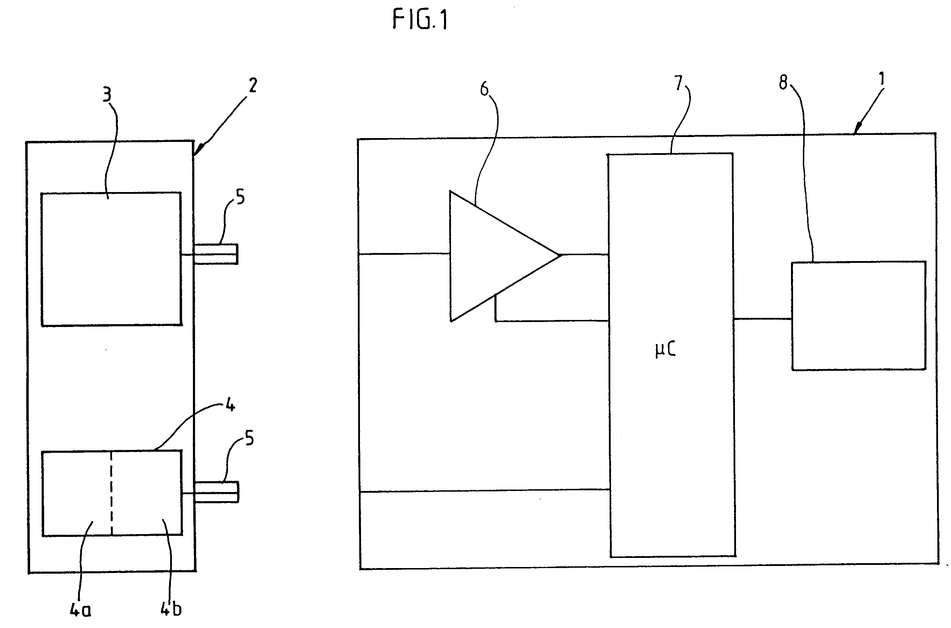

Im folgenden wird ein Ausführungsbeispiel der Erfindung anhand einer Zeichnung näher erläutert. Die Zeichnung zeigt ein Blockschaltbild für ein aus Sensor und Auswerteelektronik bestehendes Gasspurenmeßsystem. Die Auswerteelektronik ist in einem separaten Meßgerät 1 untergebracht. Der Sensor 2, der aus der Meßzelle 3 und einem elektronischen Speicher 4 besteht, kann als Austauschmodul mittels einer mehrpoligen Steckverbindung 5 auf das Meßgerät 1 aufgesteckt werden. Als Speicher wird ein nicht flüchtiger Miniaturspeicherbaustein, z.B. ein batteriegepufferter RAM oder EEPROM, verwendet. Ein Teil des Speicherbereiches 4a bleibt zweckmäßig für die vom Hersteller mitgelieferten sensorspezifischen Daten reserviert und darf daher nicht überschrieben werden, während ein anderer Teil 4b anwenderspezifische Daten aufnehmen kann.

Das Meßgerät besteht im wesentlichen aus einem Verstärker 6 für die Verstärkung des Meßsignals, einem Mikroprozessor 7 und der Anzeige 8. Der Mikroprozessor 7 dient zur Verarbeitung des Meßsignals, zur Einstellung der Verstärkung des Meßverstärkers 6, zur Ansteuerung des Speichers 4 und zur Weiterverarbeitung der darin gespeicherten Daten. Im Prinzip könnte die Kommunikation des Sensors 2 mit dem Meßgerät 1 auch über eine einzige Leitung erfolgen. Zweckmäßig sind jedoch getrennte Leitungen für die Meßzelle 3 bis zum Meßverstärker 6 und vom Speicher 4 zum Mikroprozessor 7 vorgesehen. Auf diese Weise bleibt die Meßfunktion der Meßzelle 3 auch dann bestehen, wenn der Mikroprozessor 7 mit dem Speicher 4 z.B. bei Schreib- und Leseoperationen kommuniziert (Simultanfunktion).

Der Speicher 4 wird nach der herstellerseitigen Kalibrierung mit den meßzellenspezifischen Kalibrierdaten beschrieben und bleibt unverlierbar und unverwechselbar mit der Meßzelle 3 verbunden. Nach dem Einbau des Sensormoduls 2 in das Meßgerät 1 wird der Speicher 4 vom Rechner 7 ausgelesen und automatisch eine Neukalibrierung durchgeführt. Die Kalibrierung erfolgt z.B. in der Weise, daß z.B. bei einer um 10 % höheren Empfindlichkeit der Meßzelle 3 der Verstärkungsfaktor um 10 % zurückgenommen wird, so daß die für einen vorgewählten Konzentrationsbereich bestimmte Anzeige 8 erhalten bleibt. Die Kalibrierung des Meßgerätes 1 kann automatisch bei jedem Sensorwechsel erfolgen. Auf diese Weise ist ein Sensorwechsel sehr viel schneller möglich und kann auch von ungeschultem Personal durchgeführt werden.

Beispielsweise werden vom Hersteller des Sensormoduls 2 nach der Kalibrierung der Zellentyp, der Nullwert und die Empfindlichkeit einer elektrochemischen Meßzelle 3 im Speicher 4 festgehalten. Beim Anwender werden dann diese Daten vom Meßgerät 1 mit Hilfe des Rechners 7 ausgelesen. Der Meßzellentyp (abhängig von der zu messenden Gasart) kann ebenfalls mit Hilfe der Anzeige 8 dargestellt werden. Vom Meßsignal der Meßzelle 3 wird zunächst der aus dem Speicher 4 ausgelesene Nullwert abgezogen und dann mit der ebenfalls ausgelesenen Empfindlichkeit multipliziert. Ohne jegliche Kalibrierung oder Abgleich seitens des Anwenders zeigt also das Meßgerät 1 automatisch die genaue Gaskonzentration unter Berücksichtigung der individuellen, herstellerseitigen Kalibrierung für diese spezielle Meßzelle 3 an. Die bisher übliche manuelle Anpassung und Einstellung des Meßwertes in Abhängigkeit von Zellentyp, Nullwert und Empfindlichkeit kann entfallen Dies bedeutet eine starke Reduzierung des Serviceaufwands. Außerdem ist die Gefahr einer Fehlparametrierung und damit einer Fehlfunktion des Meßgerätes ausgeschlossen. Sofern anwenderseitig eine Nachkalibrierung der Meßzelle 3 erfolgen soll, können die neuen Kalibrierdaten ebenfalls im Speicher abgelegt werden. In diesem Fall muß der Speicherbereich (4a), der die vom Hersteller mitgelieferten Daten enthält, schreibgeschützt sein.

Außer den obengenannten Parametern für die Kalibrierung können z.B. das Herstelldatum, das Verfallsdatum, die Seriennummer, der Temperaturkoeffizient, das Kalibrierdatum, der Name des Prüfers, Alarmgrenzwerte (MAK-Wert), die Integrationszeit des Meßgerätes, die Betriebsstunden die insgesamt gemessene Gasdosis im Sensormodul 2, aber auch komplexe Daten wie z.B. Temperaturgang, Linearitätsverlauf oder Druckabhängigkeit in Form von Polynomkoeffizienten gespeichert werden. Diese Daten können in bekannter Weise zur Fehlererkennung oder für eine Korrektur (z.B. mit Hilfe einer Prüfsumme) verwendet werden.

## Patentansprüche

1. Gasspurenmeßsystem, bestehend aus einem Gassensor (2) und einer Auswerteelektronik (1) mit Anzeige (8), dadurch gekennzeichnet, daß der Gassensor (2) neben der Meßzelle (3) einen elektronischen, nicht flüchtigen Speicher (4) enthält, in dem meßzellenspezifische Daten gespeichert sind und daß die Auswerteschaltung (1) einen Rechner (7) umfaßt, der die meßzellenspezifischen Daten ausliest und weiterverarbeitet.

2. Gasspurenmeßsystem nach Anspruch 1, dadurch gekennzeichnet, daß der Gassensor (2) mit der Meßzelle (3) und dem Speicher (4) als Austauschmodul ausgebildet ist.

3. Gasspurenmeßsystem nach Anspruch 1 bis 2, dadurch gekennzeichnet, daß der Speicher (4) zumindest Informationen über den Meßzellentyp hinsichtlich der zu messenden Gaskomponente, die Empfindlichkeit und die Nullpunktsverschiebung enthält.

4. Gasspurenmeßsystem nach Anspruch 3, dadurch gekennzeichnet, daß der Rechner (7) beim erstmaligen Betrieb des Gasspurenmeßsystems die Empfindlichkeit und die Nullpunktsverschiebung aus dem Speicher (4) abfragt und eine automatische Kalibrierung des Gerätes durchführt.

5. Gasspurenmeßsystem nach Anspruch 1 - 4, dadurch gekennzeichnet, daß der Gassensor (2) aus einem Austauschmodul mit einer elektrochemischen Meßzelle (3) besteht, deren Kenndaten aus dem im Austauschmodul integrierten elektronischen Speicher (4) abrufbar sind.
